# EUROPÄISCHE PATENTSCHRIFT

(11) **EP 1 334 741 B1**
(45) Veröffentlichungstag und Bekanntmachung des Hinweises auf die Patenterteilung: **12.01.2005**
(21) Anmeldenummer: 03000487.3
(22) Anmeldetag: 11.01.2003
(51) Int. Cl.: A61M 16/00

(54) **Verfahren und Vorrichtung zur Bereitstellung von Atemgas**
Method and apparatus for providing breathing air
Procédé et dispositif pour fournir de l'air respirable

(30) Priorität: 12.02.2002 DE 10205955
(43) Veröffentlichungstag der Anmeldung: 13.08.2003
(73) Patentinhaber: Weinmann Geräte für Medizin GmbH & Co. KG, 22525 Hamburg (DE)
(72) Erfinder: Feldhahn, Karl-Andreas, Dr., 22761 Hamburg (DE); Döring, Tilmann, 22459 Hamburg (DE); Ress, Thomas, 25469 Halstenbek (DE); Griefahn, Marc, 22453 Hamburg (DE)
(74) Vertreter: Klickow, Hans-Henning, Dr.-Ing.

(56) Entgegenhaltungen:
- EP-A- 0 247 365
- DE-U- 20 104 713
- US-A- 3 374 158

## Beschreibung

Die Erfindung betrifft ein Verfahren zur Bereitstellung von Atemgas, für Verwendungen außerhalb der therapeutischen Behandlung des menschlichen oder tierischen Körpers, bei dem von mindestens einem Sauerstoffgenerator erzeugter Sauerstoff einer Druckgasflasche zugeführt wird, und bei dem der Sauerstoffgenerator eine elektrolytische Zersetzung von Wasser in Wasserstoff und Sauerstoff durchführt sowie der erzeugte Sauerstoff komprimiert in die Druckgasflasche eingespeist wird.

Die Erfindung betrifft darüber hinaus eine Vorrichtung zur Bereitstellung von Atemgas, die einen Sauerstoffgenerator sowie einen Adapter zum Anschluß des Sauerstoffgenerators an eine Druckgasflasche aufweist und bei der der Sauerstoffgenerator eine Elektrolysezelle mit mindestens zwei Elektroden zur elektrolytischen Zersetzung von Wasser in Wasserstoff und Sauerstoff aufweist und bei der die Elektrolysezelle als ein Druckbehälter mit einer Innendruckbeständigkeit von mindestens 20bar ausgebildet ist.

Sauerstoffgeneratoren in einer Ausbildung als Sauerstoffkonzentratoren werden typischerweise verwendet, um stationär Patienten mit entsprechenden Krankheitssymptomen mit Atemgas zu versorgen, das eine starke Anreicherung an Sauerstoff aufweist. Typischerweise werden derartige Sauerstoffkonzentratoren im Bereich einer Wohnung des Patienten bzw. in Arztpraxen oder Krankenhäusern installiert. Für eine mobile Versorgung der Patienten werden Druckgasflaschen verwendet, die der Patient mitführen kann und mit denen der Patient in die Lage versetzt wird, zumindest bei einem Auftreten von akuten Symptomen unmittelbar selbst eine Versorgung mit einer erhöhten Sauerstoffkonzentration durchführen zu können.

Der Benutzer von derartigen Druckgasflaschen besitzt entweder selbst eine eigene Befülleinrichtung für diese Druckgasflaschen, die an einen hierfür vorgesehenen Sauerstoffkonzentrator angeschlossen werden kann, oder der Benutzer tauscht bei entsprechenden Anbietern seine geleerte Druckgasflasche gegen eine gefüllte Druckgasflasche ein.

Ebenfalls ist es bereits bekannt, für eine mobile Versorgung eines Patienten thermisch isolierte Behälter zu verwenden, in die gekühlter flüssiger Sauerstoff eingefüllt wird. Aufgrund einer kontinuierlichen Aufnahme von Umgebungswärme müssen derartige Vorrichtungen aber permanent Sauerstoff abgeben, so daß nur eine beschränkte Benutzungsdauer je Füllung vorliegt.

Generell erweist es sich bei der Befüllung von Druckgasflaschen mit komprimiertem Sauerstoff als nachteilig, daß relativ leistungsfähige Kompressoren verwendet werden müssen, um die Druckgasflaschen mit einem derart hohen Befüllungsdruck zu versehen, daß eine ausreichende Befüllmenge an Sauerstoff bereit steht. Entsprechende Kompressoren mit einem Arbeitsbereich bis zu 200bar sind zum einen teuer in der Anschaffung, darüber hinaus liegt auch ein bei vielen Anwendungen als nachteilig empfundenes Gerätevolumen vor und es besteht bei einem Gerätebetrieb ein Geräuschpegel, der insbesondere bei Heimanwendungen als störend empfunden wird.

Aus der DE 201 04 713 U ist bereits ein Verfahren zur Bereitstellung von Atemgas bekannt, bei dem von einem Sauerstoffgenerator erzeugter Sauerstoff einer Druckgasflasche zugeführt wird. Der Sauerstoffgenerator führt eine elektrolytische Zersetzung von Wasser in Wasserstoff und Sauerstoff durch. Der erzeugte Sauerstoff wird komprimiert in die Druckgasf lasche eingespeist. Darüber hinaus ist über eine Versorgungsleitung und eine Verbrauchssteuerung ein Anschluß an eine Sauerstoffbrille möglich.

Aus der US-A 3, 374, 158 ist ein weiteres System zur elektrolytischen Zersetzung von Wasser bekannt. Über eine geeignete Befeuchtung wird die Wasserkonzentration im elektrolytischen System gesteuert.

Aus der EP-A-0 247 365 ist es bereits bekannt, Behälter mit sauerstoffangereicherter Atemluft unter Verwendung von Verdichtern zu Befüllen. Der Verdichter kann über Ventile an mehrere zu füllende Sauerstoffbehälter angeschlossen werden.

Aufgabe der vorliegenden Erfindung ist es, ein Verfahren der einleitend genannten Art derart zu verbessern, daß eine hochverdichtete Befüllung der Druckgasflaschen ohne Verwendung von Hochleistungskompressoren ermöglicht wird.

Diese Aufgabe wird erfindungsgemäß dadurch gelöst, daß der Sauerstoffgeneratoren eine Befüllung der Druckgasflasche und/oder eine Atemgasversorgung für eine Sauerstoffbrille durchführen und daß mindestens zwei Sauerstoffgeneratoren parallel betrieben werden und daß der Sauerstoffgenerator zur Befüllung der Druckgasflaschen mit einem höheren Druck als der Sauerstoffgenerator zur Atemgasversorgung der Sauerstoffbrille betrieben wird.

Weitere Aufgabe der vorliegenden Erfindung ist es, eine Vorrichtung der einleitend genannten Art derart zu konstruieren, daß ein Befüllvorgang für die Druckgasflaschen bei einem geringen Geräuschpegel durchgeführt werden kann.

Diese Aufgabe wird erfindungsgemäß dadurch gelöst, daß zusätzlich zum Sauerstoffgenerator für die Befüllung der Druckgasflasche ein Sauerstoffgenerator zur Versorgung einer Sauerstoffbrille vorgesehen ist und daß der Sauerstoffgenerator zur Versorgung der Sauerstoffbrille einen niedrigeren Arbeitsdruck als der Sauerstoffgenerator zur Befüllung der Druckgasflasche aufweist.

Durch die Erzeugung des für den Befüllvorgang der Druckgasflaschen benötigten Sauerstoffes über einen Elektrolysevorgang ist es möglich, den Sauerstoff im Bereich des Sauerstoffgenerators bereits in komprimierter Form bereitzustellen, so daß entweder nur noch eine vergleichsweise geringe Nachkomprimierung erforderlich ist oder eine Nachkomprimierung sogar vollständig entfallen kann. Der technisch durch den Elektrolysevorgang bereitstellbare Druck hängt lediglich von der technisch realisierten Druckfestigkeit der verwendeten Gerätekomponenten sowie den vorgegebenen sonstigen chemischen und physikalischen Parametern bei der Durchführung des Elektrolysevorganges ab.

Grundsätzlich nimmt die Menge des bei der Elektrolyse erzeugten Sauerstoffes aufgrund einer zunehmenden Rekombination bei zunehmendem Druck ab, die technische Ausbeute kann aber durch die Vorgabe geeigneter physikalischer und chemischer Elektrolyseparameter optimiert werden. Insbesondere ist hierbei an die Konstruktion, das Material sowie die Oberflächenbeschaffenheit der Elektroden, die Vorgabe des Elektrolysestromes sowie die Elektrolysetemperatur gedacht.

Zur direkten Bereitstellung mindestens eines Teiles des für die Befüllung der Druckgasflaschen benötigten Druckes ist vorgesehen, daß die elektrolytische Zersetzung bei einem Druck oberhalb eines atmosphärischen Umgebungsdruckes durchgeführt wird.

Ein typischer Betriebsbereich wird dadurch bereitgestellt, daß die elektrolytische Zersetzung bei einem Druck im Bereich von 20bar bis 150bar durchgeführt wird.

Unter Berücksichtigung technischer Randbedingungen ist insbesondere daran gedacht, daß die elektrolytische Zersetzung bei einem Druck im Bereich von 50bar bis 100bar durchgeführt wird.

Ein vorteilhafter Kompromiß zwischen einer ergiebigen Durchführung der Elektrolyse und einem kompakten Geräteaufbau besteht darin, daß der Sauerstoff nach seiner Erzeugung im Sauerstoffgenerator und vor seiner Einspeisung in die Druckgasflasche auf ein Druckniveau oberhalb des Druckes bei der elektrolytischen Zersetzung des Wassers komprimiert wird.

Zur Gewährleistung einer langandauernden Betriebsfähigkeit der Befülleinrichtung und der Druckgasflasche trägt es bei, daß der Sauerstoff vor seiner Einspeisung in die Druckgasflasche getrocknet wird.

Eine Durchführung des Trocknungsvorganges kann beispielsweise dadurch erfolgen, daß der Feuchtigkeitsgehalt des Sauerstoffes durch Kältetrocknung vermindert wird.

Eine gerätetechnisch besonders einfache Variante besteht darin, daß der Feuchtigkeitsgehalt des Sauerstoffes durch Feuchtigkeitsabsorption vermindert wird.

Ein extrem wartungsarmer Betrieb kann dadurch erreicht werden, daß der Feuchtigkeitsgehalt des Sauerstoffes durch Membranabscheidung vermindert wird.

Zur Verminderung einer erforderlichen externen Wasserzufuhr für den Sauerstoffgenerator wird vorgeschlagen, daß bei der Trocknung des Sauerstoffes kondensierte Feuchtigkeit mindestens teilweise in den Bereich des Sauerstoffgenerators zurückgeführt wird.

Erweiterte Nutzungsmöglichkeiten werden dadurch bereitgestellt, daß der Sauerstoffgenerator sowohl eine Befüllung der Druckgasflasche als auch eine Atemgasversorgung für eine Sauerstoffbrille durchführt.

Zur Unterstützung einer Sauerstoffversorgung mit möglichst geringen Sauerstoffmengen bei gleichzeitiger Vermeidung von Sauerstoffverlusten an eine Umgebung ist vorgesehen, daß die Sauerstoffbrille über eine verbrauchssteuerung an den Sauerstoffgenerator angeschlossen wird.

Eine weitere Anwendungsvariante besteht darin, daß mindestens zwei Sauerstoffgeneratoren parallel betrieben werden und daß der Sauerstoffgenerator zur Befüllung der Druckgasflasche mit einem höheren Druck als der Sauerstoffgenerator zur Atemgasversorgung der Sauerstoffbrille betrieben wird.

Eine Dimensionierungsanpassung an konkret vorliegende Anwendungsanforderungen kann dadurch erfolgen, daß der Sauerstoffgenerator zur Befüllung der Druckgasflasche mit einer geringeren Produktionskapazität als der Sauerstoffgenerator zur Versorgung der Sauerstoffbrille betrieben wird.

Ein Notlaufbetrieb beim Ausfall einer Gerätekomponente kann dadurch erreicht werden, daß die Sauerstoffgeneratoren im Hinblick auf ihre jeweilige Funktion umschaltbar betrieben werden.

Ein weiteres Anwendungsgebiet kann dadurch erschlossen werden, daß der Sauerstoffgenerator zur Befüllung der Druckgasflasche gemeinsam mit einem Sauerstoffkonzentrator zur Atemgasversorgung der Sauerstoffbrille betrieben wird.

Zur technischen Ermöglichung einer Durchführung der Elektrolyse bei erheblichem atmosphärischen Überdruck ist vorgesehen, daß der Druckbehälter eine Druckbeständigkeit von mindestens 100bar aufweist.

Eine zusätzliche Erhöhung des Betriebsdruckes wird dadurch unterstützt, daß der Druckbehälter eine Druckbeständigkeit von mindestens 200bar aufweist

In den Zeichnungen sind Ausführungsbeispiele der Erfindung schematisch dargestellt. Es zeigen:
- Fig. 1: Eine schematische Darstellung einer Befülleinrichtung, an die ein Sauerstoffgenerator, ein Trockner, ein Adapter mit Druckminderer und eine Abgabesteuerung zur Verbindung mit einer Sauerstoffbrille sowie eine Druckgasflasche angeschlossen sind,
- Fig. 2: eine schematische Darstellung einer Elektrolysezelle mit Peripherie-Geräten zur elektrolytischen Herstellung von Sauerstoff,
- Fig. 3: eine stark vereinfachte schematische Darstellung einer Elektrolysezelle zur Veranschaulichung der Feuchtigkeitsbeladung des elektrolytisch erzeugten Sauerstoffes,
- Fig. 4: eine gegenüber Fig. 1 modifizierte Einrichtung zur Befüllung von Druckgasflaschen mit Sauerstoff und / oder zur Versorgung eines Patienten mit Sauerstoff,
- Fig. 5: eine gegenüber Fig. 4 abgewandelte Ausführungsform, bei der mindestens zwei Zellen zur elektrolytischen Herstellung von Sauerstoff verwendet sind und bei der eine Zelle komprimierten Sauerstoff erzeugt und die andere Zelle den Sauerstoff bei Umgebungsdruck abgibt,
- Fig. 6: eine gegenüber Fig. 5 abgewandelte Ausführungsform, bei der zusätzlich zur Elektrolysezelle zur Herstellung von komprimiertem Sauerstoff ein Sauerstoffkonzentrator betrieben wird, der nicht elektrolytisch arbeitet,
- Fig. 7: eine perspektivische Darstellung einer Druckgasflasche mit Druckarmaturen sowie angeschlossener Verbrauchssteuerung und Sauerstoffbrille und
- Fig. 8: eine vergrößerte Darstellung der Verbrauchssteuerung mit über einen Verbindungsschlauch angeschlossener Sauerstoffbrille sowie teilweise dargestelltem Verbindungsschlauch zum Anschluß an die Druckgasflasche.

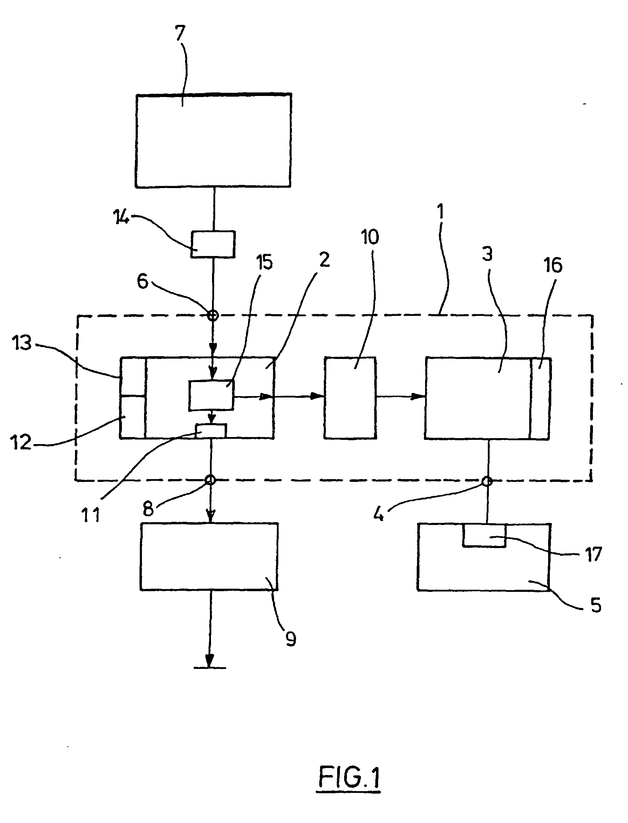

Fig. 1 zeigt eine Befülleinrichtung (1), die mit einer Steuereinrichtung (2) sowie einem Verdichter (3) versehen ist. Der Verdichter (3) ist über einen Befüllausgang (4) mit einer Druckgasflasche (5) verbindbar. Die Befülleinrichtung (1) ist über einen Atemgaseingang (6) an einen Sauerstoffgenerator (7) angeschlossen und über einen Atemgasausgang (8) kann eine Verbindung zu einer Sauerstoffbrille (9) realisiert werden. Der Anschluß des Verdichters (3) an den Sauerstoffgenerator (7) kann unter Zwischenschaltung eines Trockners (10) erfolgen.

An die Steuereinrichtung (2) ist beim dargestellten Ausführungsbeispiel ein Sensor (11) für die Erfassung eines aktuellen Verbrauches an Atemgas im Bereich der Sauerstoffbrille (9) angeschlossen. Der Sensor (11) kann beispielsweise als ein Sensor zur Erfassung einer Sauerstoffkonzentration und / oder zur Erfassung eines Volumenflusses an Atemgas ausgebildet sein. Darüber hinaus ist es auch möglich, die Steuereinrichtung (2) mit einem Einstellelement (12) zur Vorgabe eines Volumenflusses von Atemgas in Richtung auf die Sauerstoffbrille (9) auszustatten. Bei Verwendung eines derartigen Einstellelementes (12) kann der Sensor (11) als Erfassungseinrichtung für eine aktuelle Einstellung des Einstellelementes (12) realisiert sein. Darüber hinaus ist es auch möglich, auf eine sensorische Erfassung zu verzichten und lediglich Einstellvorgaben als Sollwerte auszuwerten. Der Begriff des Sensors (11) umfaßt somit auch die Übernahme eines vorgegebenen Wertes ohne Durchführung einer Messung

Beim Ausführungsbeispiel gemäß Fig. 1 ist die Befülleinrichtung (1) mit einer Anzeige (13) für mindestens einen Betriebparameter versehen. Darüber hinaus ist zwischen dem Atemgaseingang (6) und dem Sauerstoffkonzentrator (7) ein Adapter (14) angeordnet, um einen Anschluß von Sauerstoffgeneratoren (7) mit unterschiedlich gestalteten Abgabeelementen zu unterstützen.

Die Steuereinrichtung (2) ist mit einem Verteiler (15) versehen, der in Abhängigkeit von jeweiligen Steuerungsvorgaben eine Weiterleitung des Atemgases in Richtung auf die Sauerstoffbrille (9) und / oder in Richtung auf den Verdichter (3) vorgibt. Der Verdichter (3) ist mit einem Antrieb (16) ausgestattet und im Bereich der Druckgasflasche (5) ist ein Flaschenventil (17) angeordnet, um bei einer Entleerung der Druckgasflasche (5) einen jeweiligen Öffnungs- oder Schließzustand vorgeben zu können. Das Flaschenventil (17) kann bei einer Benutzung der Druckgasflasche (5) durch einen Patienten zusätzlich mit einem Druckminderer gekoppelt werden, um einen jeweiligen Verbrauchsdruck vorzugeben.

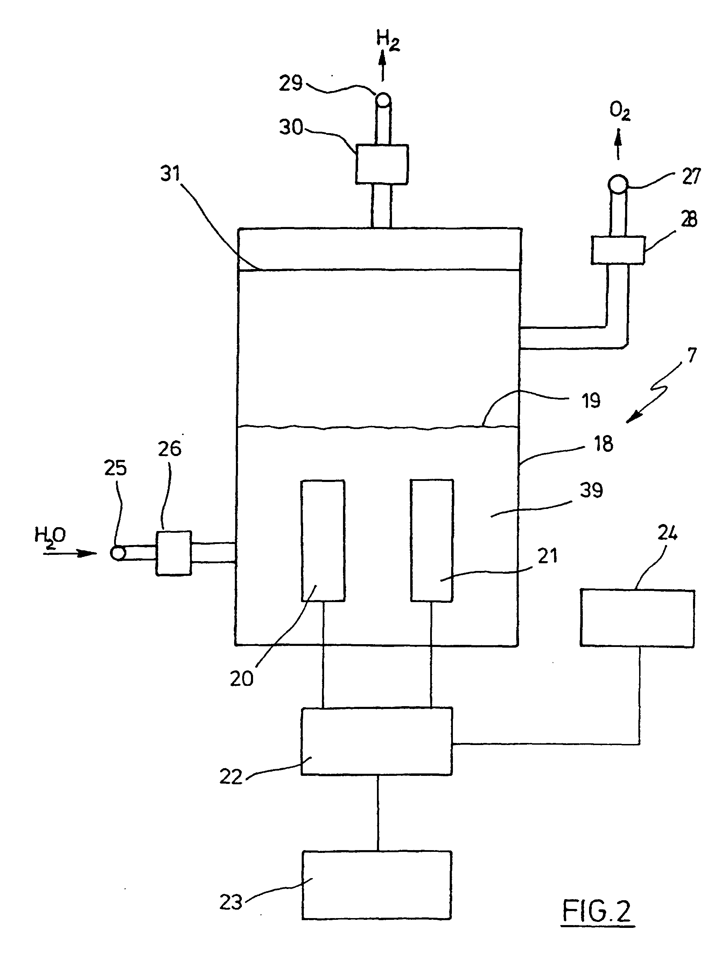

Fig. 2 zeigt einen schematischen Aufbau eines Sauerstoffgenerators (7), der elektrolytisch Sauerstoff erzeugt. Innerhalb eines Druckbehälters (18) ist bis zu einem Pegelstand (19) Wasser angeordnet. Innerhalb des Wassers sind zwei Elektroden (20, 21) positioniert, die an eine Elektrolysesteuerung (22) angeschlossen sind. Die Elektrolysesteuerung (22) ist mit einer elektrischen Energieversorgung (23) verbunden. Darüber hinaus ist die Elektrolysesteuerung (22) mit einer übergeordneten Gerätesteuerung (24) verbunden.

Der Druckbehälter (18) weist einen Flüssigkeitseinlaß (25) mit Einlaßventil (26), einen Sauerstoffauslaß (27) mit Abgabeventil (28) sowie eine Wasserstoffableitung (29) mit Auslaßventil (30) auf. Innerhalb des Druckbehälters (18) ist zur Trennung des generierten Sauerstoffes vom abzuführenden Wasserstoff ein Separator (31) angeordnet. Der Separator (31) kann beispielsweise als eine Membran ausgebildet sein, die im Bereich eines Sammelraumes (32) oberhalb des Pegelstandes (19) positionierbar ist.

Grundsätzlich ist es auch denkbar, im Bereich des Druckbehälters (18) einen als Membran ausgebildeten Trockner (10) anzuordnen. Als Membrane sind hierbei insbesondere Polymermembrane geeignet, insbesondere ist an die Verwendung einer Polymermembran aus Nafion gedacht. Ein derartiges Material kann auch zur Realisierung des Separators (31) zur Trennung von Wasserstoff und Sauerstoff verwendet werden.

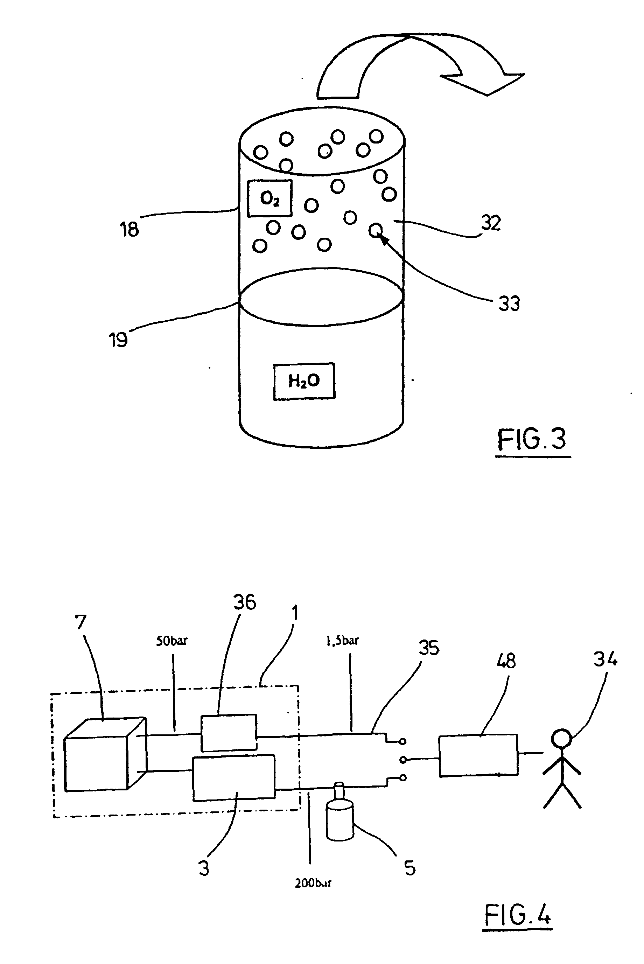

Fig. 3 zeigt schematisch nochmals den Druckbehälter (18), der bis zu einem Pegelstand (19) mit Wasser gefüllt ist. Aufgrund der elektrolytischen Herstellung des Sauerstoffes und aufgrund des perlenden Austrittes des Sauerstoffes aus dem Wasser, bei dem Feuchtigkeitspartikel (33) mitgerissen werden, liegt eine sehr hohe Feuchtigkeitssättigung im Sammelraum (32) innerhalb des Druckbehälters (18) oberhalb des Pegelstandes (19) vor. Die Vielzahl von Feuchtigkeitspartikeln (33) erweisen sich bei einer Befüllung der Druckgasflaschen (5) als nachteilig, da mit steigendem Druck des Sauerstoffes die Aufnahmekapazität für Feuchtigkeit abnimmt und hierdurch eine Kondensation stattfindet. Mit Hilfe des Trockners (10) ist es deshalb möglich, eine Kondensatbildung innerhalb der Druckgasflaschen (5) zu verhindern bzw. zumindest stark zu reduzieren.

Fig. 4 zeigt eine weitere Ausführungsform. Vom Sauerstoffgenerator (7) wird hier parallel, wahlweise oder umschaltbar sowohl eine Befüllung einer Druckgasflasche (5) durchgeführt als auch die Versorgung eines Patienten (34) unterstützt. Der Sauerstoffgenerator (7) ist hier derart dimensioniert, daß ein Elektrolysedruck von etwa 50bar realisiert ist. Zur Ermöglichung einer Befüllung der Druckgasflaschen (5) mit einem Befülldruck von ca. 200bar wird deshalb ein zusätzlicher Verdichter (3) verwendet.

Ein Anschlußschlauch (35) zur Versorgung eines Patienten (34) ist über einen Druckminderer (36) an den Sauerstoffgenerator (7) angeschlossen. Der Patient (34) kann wahlweise mit einer Verbrauchssteuerung (48) als Anschlußelement eine Verbindung zur Druckgasflasche (5) oder zum Anschlußschlauch (35) vornehmen. Im Bereich des Anschlußschlauches (35) liegt in Abhängigkeit von der Einstellung des Druckminderers ein Verbrauchsdruck vor, der typischerweise im Bereich von 1, 5bar vorgegeben ist.

Der Sauerstoffgenerator (7) ist bei der Ausführungsform gemäß Fig. 4 dafür geeignet, parallel sowohl eine Befüllung der Druckgasflasche (5) als auch eine Beatmung des Patienten (34) vorzunehmen. Grundsätzlich kann die Befülleinrichtung derart konstruiert werden, daß gleichzeitig eine Mehrzahl von Druckgasflaschen (5) mechanisch anschließbar sind.

Eine Befüllsteuerung läuft dabei typischerweise derart ab, daß die Druckgasflaschen (5) nacheinander befüllt werden, so daß bereits nach vergleichsweise kurzer Zeit auch bei einem Anschluß mehrerer Druckgasflaschen (5) eine der Druckgasflaschen (5) vollständig befüllt ist und dem Patienten (34) für eine mobile Versorgung zur Verfügung steht.

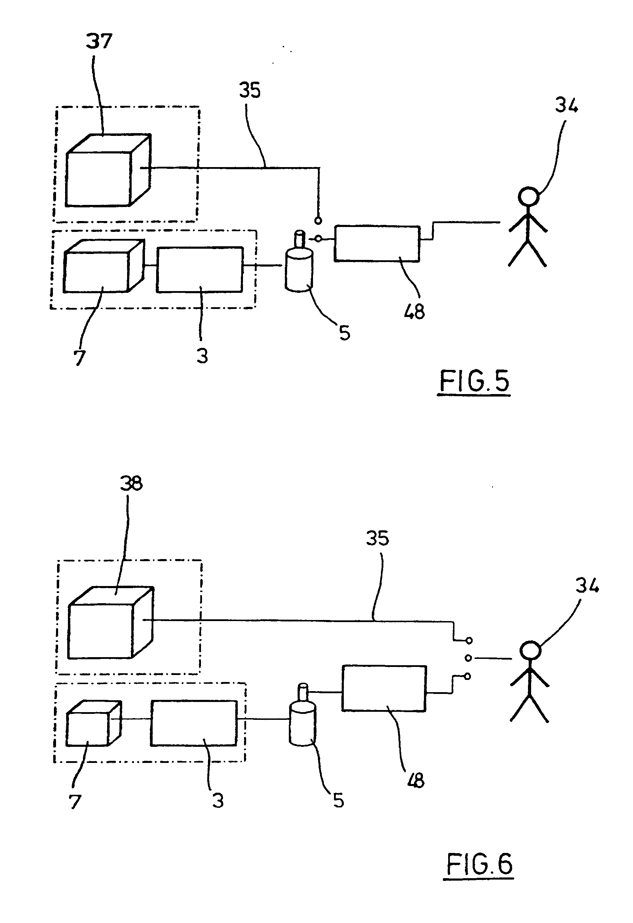

Bei der Ausführungsform gemäß Fig. 5 ist zusätzlich zum Sauerstoffgenerator (7) für die Befüllung der Druckgasflasche (5) ein zweiter Sauerstoffgenerator (37) verwendet, der ausschließlich zur direkten Atemgasversorgung des Patienten (34) vorgesehen ist. Insbesondere ist daran gedacht, den zweiten Sauerstoffgenerator (37) mit einem Druck zu betreiben, der etwa einem Umgebungsdruck entspricht. Hierdurch kann ein zusätzlicher Druckminderer (36) vermieden werden. Bei einem Betrieb des Sauerstoffgenerators (7) unterhalb des maximalen Befülldruckes der Druckgasflasche (5) wird zur Befüllung der Druckgasflasche (5) wieder ein Verdichter (3) verwendet, der die restliche Verdichtung vom Druckniveau des Sauerstoffgenerators (7) zum Befülldruck der Druckgasflasche (5) vornimmt.

Auch bei der Ausführungsform gemäß Fig. 5 kann der Patient (34) mit seiner Verbrauchssteuerung (38) wahlweise einen Anschluß an den Sauerstoffgenerator (37) oder die Druckgasflasche (5) vornehmen. Insbesondere ist bei dieser Ausführungsform daran gedacht, den Sauerstoffgenerator (37) mit einer größeren Produktionskapazität als den Sauerstoffgenerator (7) zu versehen. Die Dimensionierung des Sauerstoffgenerators (37) erfolgt hierbei derart, daß eine vollständige Deckung eines aktuellen Sauerstoffbedarfs des Patienten (34) gesichert ist. Die Kapazität des Sauerstoffgenerators (7) wird derart dimensioniert, daß die benötigte Tagesmenge für übliche mobile Verwendungen bereitgestellt wird. Eine typische Dimensionierung erfolgt dabei derart, daß eine Sauerstoffmenge bereitgestellt wird, die bei Umgebungsdruck etwa 450 Liter beträgt.

Durch eine geeignete Steuerung können Notlaufeigenschaften dadurch bereitgestellt werden, daß eine Umschaltbarkeit der Sauerstoffgeneratoren (7, 37) realisiert ist. Der Patient (34) kann somit bei einem Ausfall eines der Sauerstoffgeneratoren (7, 37) vorgeben, welche Funktion der verbleibende Sauerstoffgenerator (7, 37) durchführen soll, insbesondere, ob eine akute Sauerstoffversorgung oder eine Befüllung der Druckgasflasche (5) durchgeführt wird.

Bei der Ausführungsform gemäß Fig. 6 wird statt des zusätzlichen elektrolytischen Sauerstoffgenerators (37) ein zusätzlicher Sauerstoffkonzentrator (38) verwendet, der Umgebungsluft aufarbeitet und beispielsweise durch Anwendung der Membrantechnik Sauerstoff aus der Umgebungsluft aufkonzentriert. Aufgrund der in der Regel hohen Produktionskapazität derartiger Sauerstoffkonzentratoren kann bei dieser Ausführungsform die Verbrauchssteuerung (48) fest der Druckgasflasche (5) zugeordnet werden und eine akute Versorgung des Patienten (34) durch den Sauerstoffkonzentrator (38) kann ohne separate Verbrauchssteuerung bzw. Verbrauchsminimierung realisiert werden.

Der Trockner (10) kann in unterschiedlichen Ausführungsformen realisiert werden. Gemäß einer Ausführungsvariante erfolgt eine Kühlung des Sauerstoffes nach einem Austritt aus dem Sauerstoffgenerator (7) und vor einer Komprimierung durch den Verdichter (3). Durch die Kühlung wird der Drucktaupunkt des Sauerstoffes herabgesetzt und ein erheblicher Teil der enthaltenen Feuchtigkeit kondensiert. Das Kondensat kann abgeleitet oder in den Sauerstoffgenerator (7) zurückgeführt werden.

Ein weiteres Trocknungsprinzip kann unter Verwendung von Absorptionselementen realisiert werden, die Feuchtigkeit anlagern können. Als Trockensubstanzen können beispielsweise dehydriertes Kupfersulfat, Kieselgur oder Silikatgel verwendet werden. Eine Wasseranlagerung kann entweder durch die Oberflächenstruktur oder durch die Aufnahme von Kristallwasser erfolgen. Derartige Absorptionselemente in einer Ausführung als Trockenpatronen weisen den Vorteil auf, daß der Patient (34) selbst eine Regenerierung durch verdampfen des angelagerten Wassers vornehmen kann. Dies kann beispielsweise im heimischen Backofen erfolgen.

Bei der bereits erwähnten Realisierung des Trockners (10) über Polymermembrane, insbesondere über Membrane aus Nafion, werden typischerweise schlauchförmige Membrane eingesetzt, bei denen durch Ionenkanäle die Wassermoleküle an die Außenseite der Membrane geleitet werden. Durch die Dimensionierung der Ionenkanäle wird gewährleistet, daß ein Durchtritt von Sauerstoff nicht erfolgt.

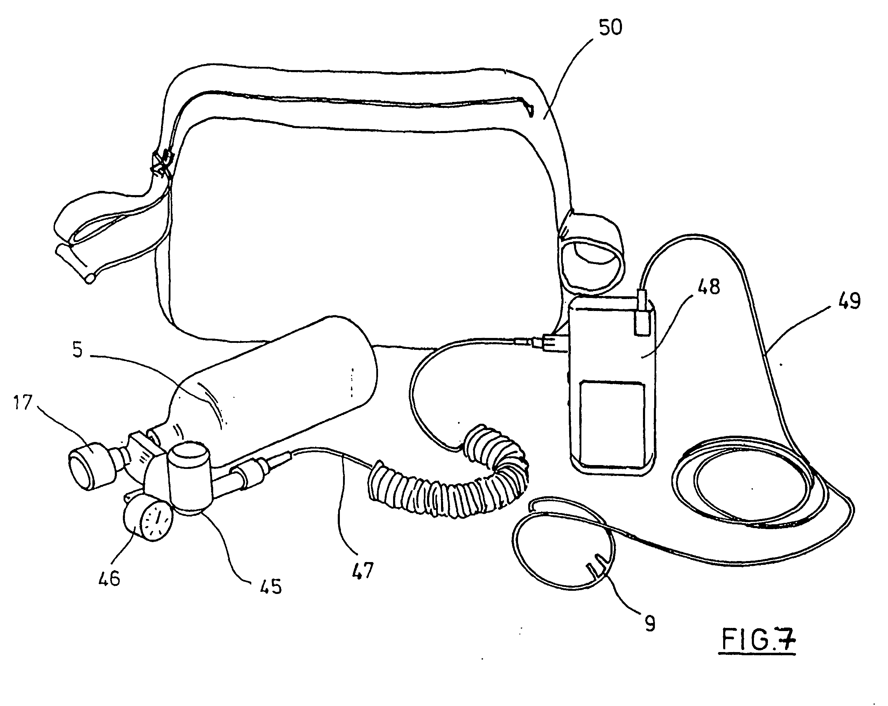

Fig. 7 zeigt die Druckgasflasche (5) mit Flaschenventil (17) sowie angeschlossener Sauerstoffbrille (9). An das Flaschenventil (17) ist ein Druckminderer (45) mit Manometer (46) zur Anzeige eines jeweiligen Verbrauchsdrucks angeschlossen. Der Druckminderer ist über einen Anschlußschlauch (47) mit der Verbrauchssteuerung (48) verbunden. Der Anschlußschlauch (47) kann als ein Spiralschlauch ausgebildet sein, um sowohl eine kompakte Gestaltung als auch unterschiedliche Verbindungslängen bereit zu stellen. An die Verbrauchssteuerung (48) ist die Sauerstoffbrille (9) über eine Versorgungsleitung (49) angeschlossen.

Der Druckminderer (45) gewährleistet, daß der Ausgangsdruck im Wesentlichen unabhängig vom Füllzustand der Druckgasflasche (5) konstant gehalten wird. Zur Vorgabe eines Volumenflusses an abgegebenem Sauerstoff kann eine Lochblende eingesetzt werden. Die Verbrauchssteuerung (48) erfaßt über einen geeigneten Sensor einen vom Patienten (34) erzeugten Einatemimpuls und steuert in Abhängigkeit von diesem erfaßten Meßwert über ein Ventil, das im Bereich der Verbrauchssteuerung (48) angeordnet ist, die Sauerstoffabgabe. Eine typische Öffnungszeit dieses Ventils beträgt etwa 40 Millisekunden je Einatmungsphase.

Zur Unterstützung einer Mobilität des Patienten (34) kann die gesamte in Fig. 7 dargestellte Einrichtung in einer Transporttasche (50) untergebracht werden. Die Transporttasche (50) ermöglicht eine hohe Mobilität des Patienten (34) und schützt die Einrichtung in einem unbenutzten Zustand gegenüber von Beschädigungen.

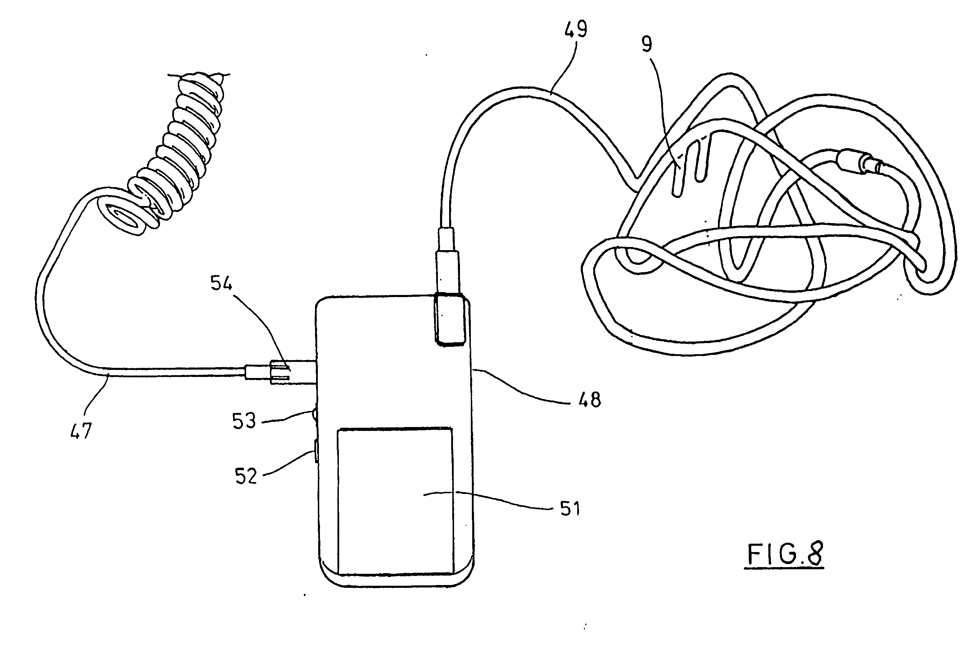

Fig. 8 veranschaulicht in etwas stärkerer Detailliertheit den Aufbau der Verbrauchssteuerung (48). Die Verbrauchssteuerung (48) ist mit einer Klappe (51) versehen, um einen Zugang zu einem Aufnahmefach für Akkumulatoren oder Batterien zu ermöglichen. Hierdurch wird eine netzunabhängige mobile Betriebsweise unterstützt. Ein Netzbetrieb oder eine Aufladung eingesetzter Akkumulatoren kann unter Verwendung einer Ladebuchse (52) durchgeführt werden. Eine Betriebsbereitschaft wird über eine Leuchtdiode (53) signalisiert. Zur Erleichterung einer Ankopplung des Anschlußschlauches (47) ist im Bereich der Verbrauchssteuerung (48) eine Schnellkupplung (54) angeordnet.

Zum Anschluß einer Sauerstoffbrille (9) an die Befülleinrichtung (1) bei gleichzeitiger Befüllung der Druckgasflasche (5) wird typischerweise zunächst der Druckminderer (45) vom Flaschenventil (17) abgeschraubt und anschließend die Druckgasflasche (5) an die Befülleinrichtung (1) angekoppelt. Der Druckminderer (45) mit Versorgungsleitung (49) wird bei einer Befüllung der Druckgasflasche (5) typischerweise nicht für eine Versorgung des Patienten verwendet, sondern der Patient verwendet hier in der Regel einen längeren Verbindungsschlauch zu einer anderen Sauerstoffbrille (9), damit ein relativ großer Bewegungsbereich für den Patienten in dessen Wohnung oder in einem anderen Aufenthaltsraum bereitgestellt wird.

## Patentansprüche

1. Verfahren zur Bereitstellung von Atemgas, für Verwendungen außerhalb der therapeutischen Behandlung des menschlichen oder tierischen Körpers, bei dem von mindestens einem Sauerstoffgenerator (7) erzeugter Sauerstoff einer Druckgasflasche (5) zugeführt wird, und bei dem der Sauerstoffgenerator (7) eine elektrolytische Zersetzung von Wasser in Wasserstoff und Sauerstoff durchführt sowie der erzeugte Sauerstoff komprimiert in die Druckgasflasche (5) eingespeist wird, **dadurch gekennzeichnet, daß** die Sauerstoffgeneratoren (7) eine Befüllung der Druckgasflasche (5) und/oder eine Atemgasversorgung für eine Sauerstoffbrille (9) durchführen und daß mindestens zwei Sauerstoffgeneratoren (7, 38) parallel betrieben werden und daß der Sauerstoffgenerator (7) zur Befüllung der Druckgasflasche (5) mit einem höheren Druck als der Sauerstoffgenerator (37) zur Atemgasversorgung der Sauerstoffbrille (9) betrieben wird.

2. Verfahren nach Anspruch 1, **dadurch gekennzeichnet, daß** die elektrolytische Zersetzung bei einem Druck oberhalb eines atmosphärischen Umgebungsdruckes durchgeführt wird.

3. Verfahren nach Anspruch 1 oder 2, **dadurch gekennzeichnet, daß** die elektrolytische Zersetzung bei einem Druck im Bereich von 20bar bis 150bar durchgeführt wird.

4. verfahren nach Anspruch 1 oder 2, **dadurch gekennzeichnet, daß** die elektrolytische Zersetzung bei einem Druck im Bereich von 50bar bis 100bar durchgeführt wird.

5. Verfahren nach einem der Ansprüche 1 bis 4, **dadurch gekennzeichnet, daß** der Sauerstoff nach seiner Erzeugung im Sauerstoffgenerator (7) und vor seiner Einspeisung in die Druckgasflasche (5) auf ein Druckniveau oberhalb des Druckes bei der elektrolytischen Zersetzung des Wassers komprimiert wird.

6. Verfahren nach einem der Ansprüche 1 bis 5, **dadurch gekennzeichnet, daß** der Sauerstoff vor seiner Einspeisung in die Druckgasflasche getrocknet wird.

7. Verfahren nach Anspruch 6, **dadurch gekennzeichnet, daß** der Feuchtigkeitsgehalt des Sauerstoffes durch Kältetrocknung vermindert wird.

8. Verfahren nach Anspruch 6, **dadurch gekennzeichnet, daß** der Feuchtigkeitsgehalt des Sauerstoffes durch Feuchtigkeitsabsorption vermindert wird.

9. Verfahren nach Anspruch 6, **dadurch gekennzeichnet, daß** der Feuchtigkeitsgehalt des Sauerstoffes durch Membranabscheidung vermindert wird.

10. Verfahren nach einem der Ansprüche 1 bis 9, **dadurch gekennzeichnet, daß** bei der Trocknung des Sauerstoffes kondensierte Feuchtigkeit mindestens teilweise in den Bereich des Sauerstoffgenerators (7) zurückgeführt wird.

11. Verfahren nach einem der Ansprüche 1 bis 10, **dadurch gekennzeichnet, daß** die Sauerstoffbrille (9) über eine Verbrauchssteuerung (48) an den Sauerstoffgenerator (7) angeschlossen wird.

12. Verfahren nach einem der Ansprüche 1 bis 11, **dadurch gekennzeichnet, daß** der Sauerstoffgenerator (7) zur Befüllung der Druckgasflasche (5) mit einer geringeren Produktionskapazität als der Sauerstoffgenerator (37) zur Versorgung der Sauerstoffbrille (9) betrieben wird.

13. Verfahren nach Anspruch 13 oder 12, **dadurch gekennzeichnet, daß** die Sauerstoffgeneratoren (7, 37) im Hinblick auf ihre jeweilige Funktion umschaltbar betrieben werden.

14. Verfahren nach einem der Ansprüche 1 bis 13, **dadurch gekennzeichnet, daß** der Sauerstoffgenerator (7) zur Befüllung der Druckgasflasche (5) gemeinsam mit einem Sauerstoffkonzentrator (38) zur Atemgasversorgung der Sauerstoffbrille (9) betrieben wird.

15. Vorrichtung zur Bereitstellung von Atemgas, die einen Sauerstoffgenerator (7) sowie einen Adapter zum Anschluß des Sauerstoffgenerators (7) an eine Druckgasflasche (5) aufweist und bei der der Sauerstoffgenerator (7) eine Elektrolysezelle (39) mit mindestens zwei Elektroden (20, 21) zur elektrolytischen Zersetzung von Wasser in Wasserstoff und Sauerstoff aufweist und bei der die Elektrolysezelle (39) als ein Druckbehälter (18) mit einer Innendruckbeständigkeit von mindestens 20bar ausgebildet ist, **dadurch gekennzeichnet, daß** zusätzlich zum Sauerstoffgenerator (7) für die Befüllung der Druckgasflasche (5) ein Sauerstoffgenerator (37) zur Versorgung einer Sauerstoffbrille (9) vorgesehen ist und daß der Sauerstoffgenerator (37) zur Versorgung der Sauerstoffbrille (9) einen niedrigeren Arbeitsdruck als der Sauerstoffgenerator (7) zur Befüllung der Druckgasflasche (5) aufweist.

16. Vorrichtung nach Anspruch 15, **dadurch gekennzeichnet, daß** der Druckbehälter (18) eine Druckbeständigkeit von mindestens 100 bar aufweist.

17. Vorrichtung nach Anspruch 15, **dadurch gekennzeichnet, daß** der Druckbehälter (18) eine Druckbeständigkeit von mindestens 200 bar aufweist

18. Vorrichtung nach einem der Ansprüche 15 bis 17, **dadurch gekennzeichnet, daß** zwischen dem Sauerstoffgenerator (7) und der Druckgasflasche (5) ein Trockner (10) angeordnet ist.

19. Vorrichtung nach Anspruch 18, **dadurch gekennzeichnet, daß** der Trockner (10) als ein Kältetrockner ausgebildet ist.

20. Vorrichtung nach Anspruch 18, **dadurch gekennzeichnet, daß** der Trockner (10) als ein Absorptionstrockner ausgebildet ist.

21. Vorrichtung nach Anspruch 18, **dadurch gekennzeichnet, daß** der Trockner (10) als ein Membrantrockner ausgebildet ist.

22. Vorrichtung nach einem der Ansprüche 15 bis 21, **dadurch gekennzeichnet, daß** der Trockner (10) über eine Kondensatrückführung mit dem Sauerstoffgenerator (7) verbunden ist.

23. Vorrichtung nach einem der Ansprüche 15 bis 22, **dadurch gekennzeichnet, daß** der Sauerstoffgenerator (7) mit einer Befülleinrichtung (1) für die Druckgasflaschen (5) verbunden ist und daß die Befülleinrichtung (1) einen Verdichter (3) zur Befüllung der Druckgasflasche (5) aufweist.

24. Vorrichtung nach einem der Ansprüche 15 bis 23, **dadurch gekennzeichnet, daß** der Sauerstoffgenerator (7) über einen Druckminderer (36) an eine Versorgungsleitung (49) zur Verbindung mit einer Sauerstoffbrille (9) angeschlossen ist.

25. Vorrichtung nach einem der Ansprüche 15 bis 24, **dadurch gekennzeichnet, daß** die Sauerstoffbrille (9) über eine Verbrauchssteuerung (48) an den Sauerstoffgenerator (7) anschließbar ist.

26. Vorrichtung nach einem der Ansprüche 15 bis 25, **dadurch gekennzeichnet, daß** der Sauerstoffgenerator (37) zur Versorgung der Sauerstoffbrille (9) eine höhere Produktionskapazität als der Sauerstoffgenerator (7) zur Befüllung der Druckgasflasche (5) aufweist.

27. Vorrichtung nach einem der Ansprüche 15 bis 26, **dadurch gekennzeichnet, daß** der Sauerstoffgenerator (7) zur Befüllung der Druckgasflasche (5) und der Sauerstoffgenerator (37) zur Versorgung der Sauerstoffbrille (9) im Hinblick auf ihre jeweilige Funktion umschaltbar angesteuert sind.

28. Vorrichtung nach einem der Ansprüche 15 bis 27, **dadurch gekennzeichnet, daß** zusätzlich zum Sauerstoffgenerator (7) der Druckgasflasche (5) ein Sauerstoffkonzentrator (38) zur Versorgung der Sauerstoffbrille (9) vorgesehen ist.

## Claims

1. Method of preparing respiratory gas for applications outside the environment of hospitalised treatment of the human or animal body, whereby oxygen generated by at least one oxygen generator (7) is delivered to a pressure cylinder (5) and whereby the oxygen generator (7) electrolytically breaks down water into hydrogen and oxygen and the generated oxygen is fed into the pressure cylinder (5) in compressed form, **characterised in that** the oxygen generators (7) fill the pressure cylinder (5) and/or a respiratory gas supply for an oxygen mask (9), and at least two oxygen generators (7, 38) are operated in parallel and the oxygen generator (7) for filling the pressure cylinder (5) is operated at a higher pressure than the oxygen generator (37) for the respiratory gas supply of the oxygen mask (9).

2. Method as claimed in claim 1, **characterised in that** the electrolytic break-down is operated at a pressure above an atmospheric ambient pressure.

3. Method as claimed in claim 1 or 2, **characterised in that** the electrolytic break-down is operated at a pressure in the range of from 20 bar to 150 bar.

4. Method as claimed in claim 1 or 2, **characterised in that** the electrolytic break-down is operated at a pressure in the range of from 50 bar to 100 bar.

5. Method as claimed in one of claims 1 to 4, **characterised in that**, after it has been generated in the oxygen generator (7) and prior to being fed into the pressure cylinder (5), the oxygen is compressed to a pressure level above the pressure at which the water was electrolytically broken down.

6. Method as claimed in one of claims 1 to 5, **characterised in that** the oxygen is dried before being fed into the pressure cylinder.

7. Method as claimed in claim 6, **characterised in that** the moisture content of the oxygen is reduced by cold-drying.

8. Method as claimed in claim 6, **characterised in that** the moisture content of the oxygen is reduced by moisture absorption.

9. Method as claimed in claim 6, **characterised in that** the moisture content of the oxygen is reduced by membrane separation.

10. Method as claimed in one of claims 1 to 9, **characterised in that** at least some of the moisture condensed when drying of the oxygen is fed back to the region of the oxygen generator (7).

11. Method as claimed in one of claims 1 to 10, **characterised in that** the oxygen mask (9) is connected to the oxygen generator (7) via a consumption controller (48).

12. Method as claimed in one of claims 1 to 11, **characterised in that** the oxygen generator (7) for filling the pressure cylinder (5) is operated at a lower production capacity than the oxygen generator (37) for supplying the oxygen mask (9).

13. Method as claimed in claim 13 or 12, **characterised in that** the oxygen generators (7, 37) are operated so that their respective functions can be switched.

14. Method as claimed in one of claims 1 to 13, **characterised in that** the oxygen generator (7) for filling the pressure cylinder (5) is operated in conjunction with an oxygen concentrator (38) for supplying the oxygen mask (9) with respiratory gas.

15. Device for preparing respiratory gas having an oxygen generator (7) and an adapter for connecting the oxygen generator (7) to a pressure cylinder (5), and in which the oxygen generator (7) has an electrolytic cell (39) with at least two electrodes (20, 21) for electrolytically breaking down water into hydrogen and oxygen, and in which the electrolytic cell (39) is provided in the form of a pressure container (18) with a capacity to withstand internal pressure of at least 20 bar, **characterised in that**, in addition to the oxygen generator (7) for filling the pressure cylinder (5), an oxygen generator (37) for supplying an oxygen mask (9) is provided and the oxygen generator (37) for supplying the oxygen mask (9) has a lower operating pressure than the oxygen generator (7) for filling the pressure cylinder (5).

16. Device as claimed in claim 15, **characterised in that** the pressure container (18) has a capacity to withstand pressure of at least 100 bar.

17. Device as claimed in claim 15, **characterised in that** the pressure container (18) has a capacity to withstand pressure of at least 200 bar.

18. Device as claimed in one of claims 15 to 17, **characterised in that** a dryer (10) is disposed between the oxygen generator (7) and the pressure cylinder (5).

19. Device as claimed in claim 18, **characterised in that** the dryer (10) is a cold-dryer.

20. Device as claimed in claim 18, **characterised in that** the dryer (10) is an absorption dryer.

21. Device as claimed in claim 18, **characterised in that** the dryer (10) is a membrane dryer.

22. Device as claimed in one of claims 15 to 21, **characterised in that** the dryer (10) is connected to the oxygen generator (7) via a condensate return line.

23. Device as claimed in one of claims 15 to 22, **characterised in that** the oxygen generator (7) is connected to a filling unit (1) for the pressure cylinder (5) and the filling unit (1) has a compressor (3) for filling the pressure cylinder (5).

24. Device as claimed in one of claims 15 to 23, **characterised in that** the oxygen generator (7) is connected via a pressure-reducer (36) to a supply line (49) for connecting to an oxygen mask (9).

25. Device as claimed in one of claims 15 to 24, **characterised in that** the oxygen mask (9) can be connected to the oxygen generator (7) via a consumption controller (48).

26. Device as claimed in one of claims 15 to 25, **characterised in that** the oxygen generator (37) for supplying the oxygen mask (9) has a higher production capacity than the oxygen generator (7) for filling the pressure cylinder (5).

27. Device as claimed in one of claims 15 to 26, **characterised in that** the oxygen generator (7) for filling the pressure cylinder (5) and the oxygen generator (37) for supplying the oxygen mask (9) are activated in such a way that their respective functions can be switched.

28. Device as claimed in one of claims 15 to 27, **characterised in that**, in addition to the oxygen generator (7) of the pressure cylinder (5), an oxygen concentrator (38) is provided in order to supply the oxygen mask (9).

## Revendications

1. Procédé pour la mise à disposition de gaz respirable, pour des utilisations en dehors du traitement thérapeutique du corps humain ou animal, dans le cadre duquel de l'oxygène produit par au moins un générateur d'oxygène (7) est conduit à une bouteille à gaz comprimé (5), et dans le cadre duquel le générateur d'oxygène (7) procède à une décomposition électrolytique d'eau en hydrogène et oxygène, et l'oxygène produit est introduit à l'état comprimé dans la bouteille à gaz comprimé (5),
**caractérisé en ce que**
les générateurs d'oxygène (7) assurent un remplissage de la bouteille à gaz comprimé (5) et / ou l'alimentation en gaz de respiration de lunettes à oxygène (9), et **en ce que** deux générateurs d'oxygène au moins (7, 38) sont exploités parallèlement, et **en ce que** le générateur d'oxygène (7) pour le remplissage de la bouteille à gaz comprimé (5) est exploité à une pression plus élevée que le générateur d'oxygène (37) pour l'alimentation en gaz respirable des lunettes à oxygène (9).

2. Procédé selon la revendication 1, **caractérisé en ce que** la décomposition électrolytique est effectuée à une pression supérieure à une pression atmosphérique ambiante.

3. Procédé selon la revendication 1 ou 2, **caractérisé en ce que** la décomposition électrolytique est effectuée à une pression située dans une plage de 20 bars à 150 bars.

4. Procédé selon la revendication 1 ou 2, **caractérisé en ce que** la décomposition électrolytique est effectuée à une pression supérieure, située dans un domaine de 50 bar à 100 bar.

5. Procédé selon l'une des revendications 1 à 4, **caractérisé en ce que** l'oxygène, après sa production dans le générateur d'oxygène (7) et avant son introduction dans la bouteille à gaz comprimé (5) est comprimé à un niveau de pression supérieur à la pression lors de la décomposition électrolytique de l'eau.

6. Procédé selon l'une des revendications 1 à 5, **caractérisé en ce que** l'oxygène est séché avant son introduction dans la bouteille à gaz comprimé.

7. Procédé selon la revendication 6, **caractérisé en ce que** le taux d'humidité de l'oxygène est réduit par séchage par réfrigération.

8. Procédé selon la revendication 6, **caractérisé en ce que** le taux d'humidité de l'oxygène est réduit par absorption de l'humidité.

9. Procédé selon la revendication 6, **caractérisé en ce que** le taux d'humidité de l'oxygène est réduit par séparation par membrane.

10. Procédé selon l'une des revendications 1 à 9, **caractérisé en ce que**, lors du séchage de l'oxygène, de l'humidité condensée est ramené au moins partiellement dans la région du générateur d'oxygène (7).

11. Procédé selon l'une des revendications 1 à 10, **caractérisé en ce que** les lunettes à oxygène (9) sont raccordées au générateur d'oxygène (7) par l'intermédiaire d'une commande de consommation (48).

12. Procédé selon l'une des revendications 1 à 11, **caractérisé en ce que** le générateur d'oxygène (7) pour le remplissage de la bouteille à gaz comprimé (5) est exploité avec une capacité de production plus faible que le générateur d'oxygène alimentant les lunettes à oxygène (9).

13. Procédé selon l'une des revendications 11 ou 12, **caractérisé en ce que** les générateurs d'oxygène (7, 37) peuvent être commutés quant à leur fonction respective.

14. Procédé selon l'une des revendications 1 à 13, **caractérisé en ce que** le générateur d'oxygène (7) pour le remplissage de la bouteille à gaz comprimé (5) est exploité en commun avec un concentrateur d'oxygène (38) pour l'alimentation en gaz de respiration des lunettes à oxygène (9).

15. Dispositif pour la mise à disposition de gaz de respiration, qui présente un générateur d'oxygène (7) ainsi qu'un adaptateur pour le raccordement du générateur d'oxygène (7) à une bouteille à gaz comprimé (5) et dans lequel le générateur d'oxygène (7) présente une cellule d'électrolyse (39) avec au moins deux électrodes (20, 21) pour la décomposition électrolytique de l'eau en hydrogène et oxygène, et dans lequel la cellule d'électrolyse (39) est conçue en tant que réservoir sous pression (18) avec une résistance à la pression d'au moins 20 bars, **caractérisé en ce que**, pour l'alimentation de lunettes à oxygène, un générateur d'oxygène (37) est prévu en plus du générateur d'oxygène (7) pour le remplissage de la bouteille à gaz comprimé (5), et **en ce que** ledit générateur d'oxygène (37) pour l'alimentation des lunettes à oxygène (9) présente une pression de service plus faible que celle du générateur d'oxygène (7) pour le remplissage de la bouteille à gaz comprimé (5).

16. Dispositif selon la revendication 15, **caractérisé en ce que** le réservoir sous pression (18) présente une résistance à la pression d'au moins 100 bars.

17. Dispositif selon la revendication 15, **caractérisé en ce que** le réservoir sous pression (18) présente une résistance à la pression d'au moins 200 bars.

18. Dispositif selon l'une des revendications 15 à 17, **caractérisé en ce qu'**un séchoir (10) est disposé entre le générateur d'oxygène (7) et la bouteille à gaz comprimé (5).

19. Dispositif selon la revendication 18, **caractérisé en ce que** le séchoir (10) est conçu sous la forme d'un séchoir par réfrigération.

20. Dispositif selon la revendication 18, **caractérisé en ce que** le séchoir (10) est conçu sous la forme d'un séchoir par absorption.

21. Dispositif selon la revendication 18, **caractérisé en ce que** le séchoir (10) est conçu sous la forme d'un séchoir à membrane.

22. Dispositif selon l'une des revendications 15 à 21, **caractérisé en ce que** le séchoir est relié au générateur d'oxygène (7) par l'intermédiaire d'un système de recyclage du produit de condensation.

23. Dispositif selon l'une des revendications 15 à 22, **caractérisé en ce que** le générateur d'oxygène (7) est relié à un dispositif de remplissage (1) pour la bouteille à gaz comprimé (5) et **en ce que** ledit dispositif de remplissage (1) présente un compresseur pour le remplissage de la bouteille à gaz comprimé (5).

24. Dispositif selon l'une des revendications 15 à 23, **caractérisé en ce que** le générateur d'oxygène (7) peut être raccordé à une conduite d'alimentation (49) par l'intermédiaire d'un réducteur de pression (36) pour le raccordement à des lunettes à oxygène (9).

25. Dispositif selon l'une des revendications 15 à 24, **caractérisé en ce que** les lunettes à oxygène (9) peuvent être raccordées au générateur d'oxygène (7) par l'intermédiaire d'une commande de consommation (48).

26. Dispositif selon l'une des revendications 15 à 25, **caractérisé en ce que** le générateur d'oxygène (37) pour l'alimentation des lunettes à oxygène (9) présente une capacité de production plus élevée que le générateur d'oxygène (7) pour le remplissage de la bouteille à gaz comprimé (5).

27. Dispositif selon l'une des revendications 15 à 26, **caractérisé en ce que** le générateur d'oxygène (7) pour le remplissage de la bouteille à gaz comprimé (5) et. le générateur d'oxygène (37) pour l'alimentation des lunettes à oxygène (9) peuvent être commutés quant à leur fonction respective.

28. Dispositif selon l'une des revendications 15 à 27, **caractérisé en ce que**, pour l'alimentation des lunettes à oxygène (9), un concentrateur d'oxygène (38) est prévu en plus du générateur d'oxygène (7) de la bouteille à gaz comprimé (5).
